# EUROPÄISCHE PATENTSCHRIFT

(11) **EP 0 455 929 B1**
(45) Veröffentlichungstag und Bekanntmachung des Hinweises auf die Patenterteilung: **03.05.1995**
(21) Anmeldenummer: 91100072.7
(22) Anmeldetag: 02.01.1991
(51) Int. Cl.: A61F 2/42

(54) **Gelenkteilprothese, insbesondere für ein Fingergelenk**
Joint part prosthesis, especially for a finger joint
Partie d'une articulation de prothèse, notamment pour une articulation de doigt

(30) Priorität: 11.05.1990 DE 9005372 U
(43) Veröffentlichungstag der Anmeldung: 13.11.1991
(73) Patentinhaber: THERA Patent GmbH & Co. KG Gesellschaft für industrielle Schutzrechte, D-82229 Seefeld (DE)
(72) Erfinder: Wilhelm, K., Prof. Dr., W-8000 München 2 (DE); Micko, Helmut, W-8031 Gilching (DE); Zöllner, Werner, Dr., W-8031 Oberpfaffenhofen (DE); Stefan, Klaus-Peter, Dr., W-8031 Seefeld (DE)
(74) Vertreter: Strehl Schübel-Hopf Groening & Partner

(56) Entgegenhaltungen:
- AT-B- 333 943
- DE-A- 2 424 537
- US-A- 4 276 660

## Beschreibung

Eine Fingergelenkprothese zum Ersatz des proximal- oder des distal-interphalangealen (PIP- bzw. DIP-)Fingergelenks ist aus US-A-4,725,280 bekannt. Der proximale Gelenkteil wird dort von einem aus Kunststoff oder einer Chrom-Molybdän-Legierung bestehenden schalenartigen Bauteil gebildet, das auf das entsprechend vorbereitete Knochenende des Finger-Grund- oder Mittelgliedes aufgesetzt wird. Der distale Gelenkteil besteht aus Kunststoff und umfaßt einen eine Lagerfläche für den anderen Prothesenteil aufweisenden Lagerkörper und einen in den Markraum des Fingergelenkknochens einzubettenden Schaft.

Bei dem distalen Gelenkprothesenteil ist der Lagerkörper als massiver Kopf ausgebildet und bedingt daher eine erhebliche Resektion des Knochens. Demgegenüber ist bei dem schalenförmig gestalteten und auf das betreffende Knochenende aufgeschobenen proximalen Gelenkprothesenteil die dauerhafte Fixierung problematisch. Ferner ist die bekannte Gelenkprothese hinsichtlich ihrer beiden miteinander zusammenwirkenden Lagerflächen so gestaltet, daß in jedem Fall der Ersatz des gesamten Gelenks erforderlich ist.

Aus DE-C-976 768 ist ein Gelenkprothesenteil mit den im ersten Teil des Anspruchs 1 angegebenen Merkmalen bekannt. Der Lagerkörper der insgesamt aus Kunststoff bestehenden Prothese ist dort als verhältnismäßig dünnwandige Schale ausgebildet und mit einem Schaft versehen, der schräg durch den Knochen verläuft oder so gekrümmt ist, daß er an einer von der Gelenkfläche entfernten Stelle aus dem Knochen austritt und dort in den kompakten äußeren Knochenbereich eingreift. Diese Art der Verankerung führt zu einer unerwünschten Beschädigung und Schwächung des Knochens.

Der Erfindung liegt die Aufgabe zugrunde, eine Prothese, insbesondere für ein Fingergelenk, anzugeben, die mit minimaler Knochenresektion auskommt, trotzdem eine sichere Verankerung im Knochen gewährleistet und es gestattet, in Fällen, in denen ein Gelenkteil noch funktionsfähig ist, nur den entsprechenden anderen Teil zu ersetzen.

Die erfindungsgemäße Lösung dieser Aufgabe ist im Patentanspruch 1 angegeben.

Durch Gestaltung des jeweiligen Lagerkörpers als dünnwandiges, aber hochfestes metallisches Schalenelement zielt die Erfindung darauf ab, im wesentlichen nur die Lagerfläche zu ersetzen, wozu bei der spezifizierten Dicke von höchstens etwa 1 mm in der Regel die Entfernung allein des Gelenksknorpels ausreicht. Gleichzeitig sorgt der an der Rückseite dieser Schale angebrachte und im Markraum des Knochens zu verankernde Schaft in Verbindung mit der im Anspruch 1 angegebenen Rauhigkeit der hinteren Schalenfläche für einen festen und dauerhaften Sitz und eine sichere Haftung des Prothesenteils an dem verankernden Knochenzement. Da die Lagerfläche der erfindungsgemäßen Gelenkteilprothese der natürlichen Form des zu ersetzenden Gelenkteils nachgebildet ist, wird es ferner möglich, nur einen Gelenkteil zu ersetzen und den anderen natürlichen Gelenkteil beizubehalten, soweit dieser wie oft bei Unfallschäden noch funktionsfähig ist.

Vorteilhafte Weiterbildungen der Erfindung sind in den Unteransprüchen gekennzeichnet. Dabei bezieht sich die Maßnahme des Anspruchs 3 auf eine gute Gleitfähigkeit der Lagerflächen gegeneinander. Die Maßnahmen der Ansprüche 4 und 5 dienen einer möglichst dauerhaften Verankerung des jeweiligen Prothesenschafts im betreffenden Knochenhohlraum unter Verwendung eines Knochenzements. Die Merkmale der Ansprüche 6 und 7 sind vom Standpunkt hoher Beweglichkeit, insbesondere maximaler Beugung, von Vorteil.

Ein Ausführungsbeispiel der Erfindung wird nachstehend anhand der Zeichnung näher erläutert. Darin zeigen
- Figur 1 und 2: Seitenansichten eines proximalen und eines distalen Gelenkprothesenteils, jeweils gesehen von dorsal,
- Figur 2A: einen Längsschnitt durch den distalen Gelenkprothesenteil in einer zur Zeichenebene der Figur 2 parallelen Schnittebene,
- Figur 3 und 4: Längsschnitte durch die beiden Prothesenteile in der Symmetrieebene X-X der Figuren 1 und 2, und
- Figur 5 und 6: Aufsichten auf die Prothesenteile, jeweils in Richtung der Achse des Prothesenschaftes.

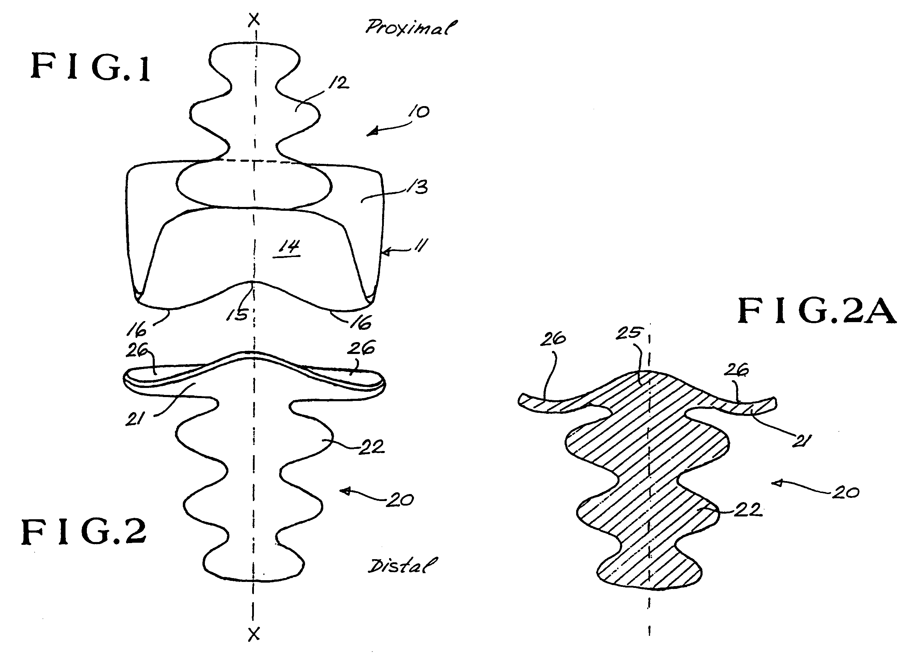

Der in Figur 1, 3 und 5 gezeigte, einen Gelenkkopf bildende proximale Gelenkprothesenteil 10 besteht aus einem Lagerkörper in Form eines Schalenelements 11 und einem an dessen Innenseite angeformten Schaft 12.

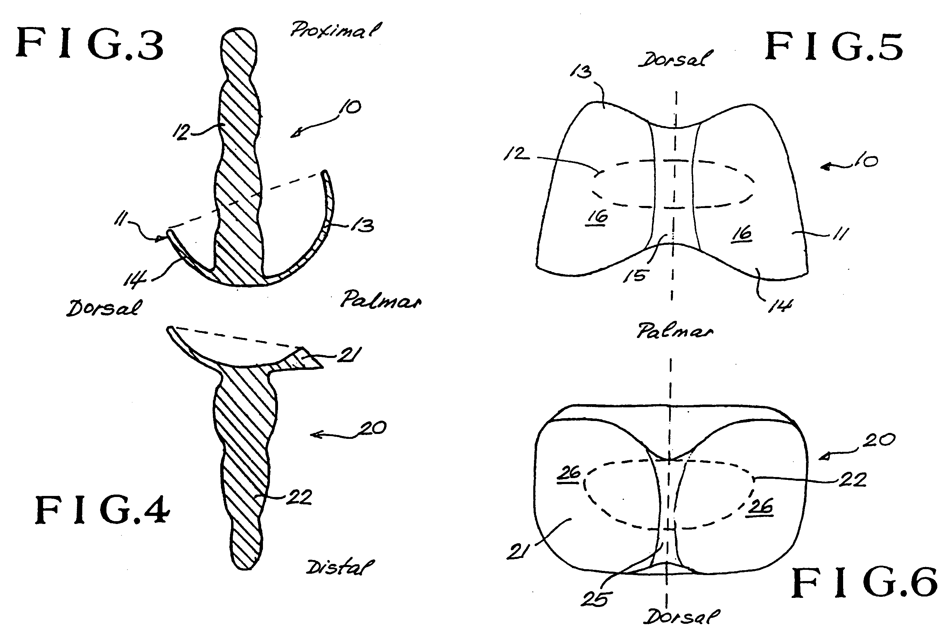

In dem Längsschnitt nach Figur 3 beschreibt das Schalenelement 11 im wesentlichen einen Halbkreisbogen, wobei der Schaft 12 dorsal versetzt exzentrisch an der Schaleninnenseite angeformt ist, so daß eine palmar verlängerte Schürze 13 entsteht, die einen entsprechend großen Beugungswinkel der Fingergelenkprothese von mindestens 90°, vorzugsweise etwa 100°, zuläßt. Wie aus Figur 1 und 5 ersichtlich, ist ferner die palmare Schürze 13 breiter als der dorsale Teil 14 des Schalenelements 11.

Die dem anderen Gelenkprothesenteil zugewandte Lagerfläche des Schalenelements 11 weist eine in der Symmetrieebene X-X verlaufende verrundete Vertiefung 15 auf, die beidseitig lateral in konvexe Flächenbereiche 16 übergeht. Insgesamt ist die Lagerfläche derjenigen eines menschlichen Fingergelenks anatomisch in idealisierter Form nachgebildet und läßt ähnlich wie ein natürliches Fingergelenk außer der Beugebewegung kleine Lateralbewegungen relativ zu dem anderen Gelenkteil zu.

Die in dem Längsschnitt nach Figur 3 gezeigte Dicke der Lagerschale 11 beträgt höchstens 1 mm und ist vorzugsweise nicht größer als 0,5 mm. Die Lagerfläche ist hochglanzpoliert und weist eine mittlere Rauhigkeit von maximal 5 »m, vorzugsweise nicht mehr als 2,5 »m auf. Demgegenüber beträgt die mittlere Rauhigkeit der Innenseite der Lagerschale 11 mehr als 5 »m und höchstens 250 »m mit einem bevorzugten Bereich von 10 bis 50 »m.

Die Lagerfläche weist abgerundete Kanten und Ecken auf, um Sehnen und Bänder nicht zu beanspruchen oder gar zu verletzen. Die Hochglanzpolierung dient zur Erzielung einer möglichst hohen Gleitfähigkeit bezüglich der gegenüberliegenden Lagerfläche.

Der Schaft 12 hat eine von den Flächen mehrerer einander teilweise durchdringender Ellipsoide oder abgeflachter Kugeln gebildete Gestalt, wobei das Verhältnis der jeweiligen aus Figur 1 ersichtlichen längsten Achse dieser Querschnittsform zu der aus Figur 3 ersichtlichen kürzesten Achse in der gleichen Querschnittsebene nicht größer ist als etwa 6, vorzugsweise 2. Diese Form gewährleistet eine gute Ausnutzung des im Querschnitt elliptischen Markraums des Fingerknochens und verhindert gleichzeitig eine Relativdrehung zwischen Prothese und Knochen.

Die Oberfläche des Schafts 12 ist mit einer mittleren Rauhigkeit von größer als etwa 5 »m bis hinauf zu etwa 500 »m mit einem bevorzugten Bereich von 10 bis 100 »m aufgerauht. Vorzugsweise weisen Schaftfläche und Lagerschaleninnenseite gleiche Rauhigkeitswerte auf.

Die so aufgerauhten Oberflächen des Schaftes 12 und der Innenseite des Schalenelements 11 können zur weiteren Verbesserung der Verbindung zwischen Zement und Prothese weiter behandelt, etwa mit einer keramischen Zwischenschicht versehen sein, die ihrerseits zusätzlich silanisiert sein kann. Dabei ist die Verwendung von methacrylfunktionellen Silanen dann zweckmäßig, wenn ein Zement auf Methacrylatbasis verwendet wird. Als Zement zur Verankerung des Schafts 12 im Knochenhohlraum sowie zur Fixierung der Innenseite des Schalenelements 11 eignen sich insbesondere Glasionomerzemente, d.h. Zemente auf der Basis eines säurelöslichen Glases und einer polymeren Polysäure.

Das Prothesenteil 10 selbst besteht aus einem Metall hoher Festigkeit, um angesichts der geringen Dicke des Schalenelements 11 ausreichende Stabilität und Abriebfestigkeit zu erzielen. Gut geeignet sind Titan oder Titanlegierungen, Edelstahllegierungen, beispielsweise auf Chrom-Kobalt-Molybdän-Basis, Edelmetallegierungen auf Silber-Palladium-Basis oder solche mit hohem Goldgehalt. Die Herstellung erfolgt vorzugsweise durch Gießen; möglich ist auch eine Herstellung durch Pressen, Fräsen oder Erodieren, wobei auch Galvanisierverfahren zur Anwendung kommen.

Der in Figur 2, 2A, 4 und 6 gezeigte, eine Gelenkbasis bildende distale Gelenkprothesenteil 20 besteht ebenfalls aus einem Schalenelement 21 und einem an dessen Rückseite angeformten Schaft 22. In dem in Figur 4 gezeigten Längsschnitt beschreibt das Schalenelement 21 etwa einen Viertelkreisbogen, wobei der Schaft palmar versetzt exzentrisch angeformt ist. Wie sich aus dem Längsschnitt nach Figur 2A und insbesondere aus der Aufsicht der Figur 6 ergibt, weist das Schalenelement 21 eine in der Symmetrieebene X-X verlaufende abgerundete Erhöhung 25 auf, die beidseitig lateral in konkave Flächenbereiche 26 übergeht.

Die Oberflächenbeschaffenheiten der dem proximalen Gelenkprothesenteil 10 zugewandten Lagerfläche des Schalenelements 21 einerseits sowie der Rückseite des Schalenelements 21 und des Schafts 22 andererseits entsprechen den oben beschriebenen Gegebenheiten des proximalen Gelenkprothesenteils 10. Das gleiche gilt hinsichtlich der Gesamtform des Schafts 22, des Werkstoffs, des Herstellverfahrens und des Zements zur Verankerung in dem betreffenden Fingergelenksknochen.

### Beispiel

Nach Fertigung eines Urmodells von Lagerfläche und Schaft in Kunststoff oder Gips wurde eine Abformung der Lagerfläche mit dem von der Firma ESPE unter der Bezeichnung REPROGUM im Handel erhältlichen Material durchgeführt. Die Abformung wurde mit einem Modellierkunststoff (dem von der Firma ESPE unter der Bezeichnung VISIO-FORM vertriebenen Material) ausgegossen, und das so erhaltene Kunststoffmodell wurde mittels Korundpapier genau nachgearbeitet. Das Urmodell des Schafts wurde ebenfalls mit dem von der Firma ESPE unter der Bezeichnung PERMAGUM vertriebenen Material ausgeformt, und in dieser Form wurde ein Kunststoffmodell aus Methylmethacrylat (dem von der Firma GC unter der Bezeichnung PATTERN RESIN vertriebenen Material) hergestellt, das anschließend ebenfalls mit Hilfe von Korundpapier nachgearbeitet wurde. Lagerfläche und Schaft wurden mittels einen Cyanacrylatklebers und Wachs (dem von der Firma SCHULER-DENTAL unter der Bezeichnung TELESKOP-WACHS vertriebenen Material) lagerichtig aneinander fixiert.

Nach zahntechnischen Verfahren wurden die Gußmodelle zusammen mit einem "verlorenen Kopf" auf einen Muffelsockel gestiftelt, aufgewachst und eingebettet. Die Muffel wurde vorgewärmt und mit einer Gußlegierung (dem von der Firma KRUPP unter der Bezeichnung WISIL vertriebenen Werkstoff, bei dem es sich um eine 60 Gew-% Cr, 30 Gew-% Co und 5 Gew-% Mo enthaltende Legierung handelt) ausgegossen. Nach Ausbettung wurde der Rohguß mittels Sandstrahlen gereinigt, das Gußobjekt (die Prothese) abgetrennt, scharfe Kanten verrundet, die Retentionsflächen (Schaft und Rückseite des Schalenelements) mit Sand einer Korngröße von 250 »m sandgestrahlt (mittlere Rauhigkeit 18 »m) und die Lagerfläche hochglanzpoliert (mittlere Rauhigkeit 1,3 »m).

Im Rahmen einer kontrollierten klinischen Studie wurden fünf Patienten PIP-Prothesen implantiert. In drei Fällen wurde Caput (phalangeale Seite) und Basis (distale Seite) implantiert. In einem Fall wurde lediglich die phalangeale, in einem weiteren Fall lediglich die distale Seite ersetzt.

Zum Einsetzen der Prothesen war nur eine minimale Resektion der Gelenkflächen (mittels oszillierender Säge) nötig. Anschließend wurde ein angemischter Glasionomerzement (dem von der Firma Ionos unter Bezeichnung V-O CEM vertriebenen Material) eingebracht und die Prothese eingesetzt. Nach Aushärten des Zements wurden die Zementüberschüsse entfernt und die Operation durch Wundverschluß usw. beendet.

In allen fünf Fällen war die postoperative Beweglichkeit auch nach einem Zeitraum von zehn Monaten noch hervorragend. Die operierten Gelenke waren bis zu einem Winkel von 90° beugungsfähig.

## Patentansprüche

1. Gelenkteilprothese, insbesondere für ein Fingergelenk, mit einem eine Lagerfläche aufweisenden Lagerkörper (11; 21) und einem in den Knochen einzubettenden Schaft (12; 22), wobei der Lagerkörper als dünnwandiges Schalenelement (11; 21) mit einer der natürlichen Form nachgebildeten Lagerfläche ausgebildet ist, dadurch gekennzeichnet, daß das Schalenelement (11; 21) aus hochfestem metallischem Werkstoff mit einer Dicke von höchstens 1 mm geformt ist, an seiner Lagerfläche eine maximale mittlere Rauhigkeit von 5 »m, vorzugsweise 2,5 »m, und an seiner hinteren Fläche eine Rauhigkeit von 5 »m bis 250 »m, vorzugsweise 10 bis 50 »m, aufweist.

2. Prothese nach Anspruch 1, dadurch gekennzeichnet, daß die Dicke des Schalenelements (11; 21) höchstens 0,5 mm beträgt.

3. Prothese nach Anspruch 1 oder 2, dadurch gekennzeichnet, daß die Lagerfläche des Schalenelements (11; 21) hochglanzpoliert und seine dem Knochen zugewandte hintere Fläche aufgerauht ist.

4. Prothese nach einem der Ansprüche 1 bis 3, dadurch gekennzeichnet, daß der Schaft (12; 22) eine von den Oberflächen mehrerer einander teilweise durchdringender Ellipsoide oder abgeflachter Kugeln gebildete Form aufweist, wobei im Querschnitt das Verhältnis der längsten zur kürzesten Achse höchstens 6, vorzugsweise 2, beträgt.

5. Prothese nach einem der Ansprüche 1 bis 4, dadurch gekennzeichnet, daß die Oberfläche des Schafts (12; 22) eine mittlere Rauhigkeit von 5 »m bis 500 »m, vorzugsweise 10 bis 100 »m, aufweist.

6. Prothese nach einem der Ansprüche 1 bis 5, dadurch gekennzeichnet, daß das den Gelenkkopf eines proximalen Gelenkteils (10) bildende Schalenelement (11) im dorsal-palmaren Längsschnitt (Figur 3) etwa einen Halbkreisbogen beschreibt und der Schaft (12) dorsal versetzt exzentrisch angebracht ist.

7. Prothese nach einem der Ansprüche 1 bis 6, dadurch gekennzeichnet, daß das die Gelenkbasis eines distalen Gelenkteils (20) bildende Schalenelement (21) im dorsal-palmaren Längsschnitt (Figur 4) etwa einen Viertelkreisbogen beschreibt und der Schaft (22) palmar versetzt exzentrisch angebracht ist.

## Claims

1. A prosthetic joint member, particularly for a finger joint, comprising a bearing body (11; 21) having a bearing surface and a shaft (12; 22) to be embedded in the bone, the bearing body being formed as a thin-walled shell portion (11; 21) shaped after the natural form, characterized in that the shell portion (11; 21) is formed of high-strength metallic material having a maximum thickness of 1 mm, with its bearing surface having a maximum average roughness of 5 »m, preferably 2.5 »m, and its rear surface having a roughness of 5 »m to 250 »m, preferably 10 to 50 »m.

2. The prosthesis of claim 1, characterized in that the shell portion (11; 21) has a maximum thickness of 0.5 »m.

3. The prosthesis of claim 1 or 2, characterized in that the bearing surface of the shell portion (11; 21) is mirror-polished and the rear surface facing the bone is roughened.

4. The prosthesis of any one of claims 1 to 3, characterized in that the shaft (12; 22) has a shape formed by ellipsoids or flattened spheres which partly penetrate each other, the maximum ratio, in cross-section, of the longest axis to the shortest axis being 6, preferably 2.

5. The prosthesis of any one of claims 1 to 4, characterized in that the surface of the shaft (12; 22) has an average roughness of 5 »m to 500 »m, preferably 10 to 100 »m.

6. The prosthesis of any one of claims 1 to 5, characterized in that the shell portion (11) constituting the joint head of a proximal joint member (10) forms, in a dorsal-palmar longitudinal section (Figure 3), approximately a semi-circular arc, the shaft (12) being connected at a location eccentrically offset in the dorsal direction.

7. The prosthesis of any one of claims 1 to 6, characterized in that the shell portion (21) constituting the joint base of a distal joint member (20) forms, in a dorsal-palmar longitudinal section (Figure 4) approximately a quarter-circular arc, the shaft (22) being connected at a location eccentrically offset in the palmar direction.

## Revendications

1. Prothèse d'élément d'articulation, en particulier pour une articulation de doigt, comprenant un support (11; 21) muni d'une surface d'appui, et une tige (12; 22) à encastrer dans l'os, le support étant réalisé sous la forme d'un élément de coque (11; 21) à paroi mince avec une surface d'appui copiant la forme naturelle, caractérisée en ce que l'élément de coque (11; 21) est constitué d'un matériau métallique à résistance élevée d'une épaisseur maximale de 1 mm, que sa surface d'appui présente une rugosité moyenne maximale de 5 »m, de préférence de 2,5 »m, et que sa surface arrière présente une rugosité moyenne de 5 »m à 250 »m, de préférence de 10 à 50 »m.

2. Prothèse selon la revendication 1, caractérisée en ce que l'épaisseur de l'élément de coque (11; 21) ne dépasse pas 0,5 mm.

3. Prothèse selon l'une des revendications 1 ou 2, caractérisée en ce que la surface d'appui de l'élément de coque (11; 21) est polie spéculaire et que sa surface postérieure tournée vers l'os est rendue rugueuse.

4. Prothèse selon l'une des revendications 1 à 3, caractérisée en ce que la tige (12; 22) présente une forme constituée par les surfaces de plusieurs ellipsoïdes ou sphères aplaties qui s'interpénètrent partiellement, dans la section transversale, le rapport entre l'axe le plus long et l'axe le plus court étant égal à 6, de préférence égal à 2.

5. Prothèse selon l'une des revendications 1 à 4, caractérisée en ce que la surface de la tige (12; 22) présente une rugosité moyenne de 5 »m à 500 »m, de préférence de 10 à 100 »m.

6. Prothèse selon l'une des revendications 1 à 5, caractérisée en ce que l'élément de coque (11) qui forme la tête d'articulation d'un élément d'articulation proximal (10) décrit sensiblement, en coupe longitudinale dorsale-palmaire (fig. 3), un demi-cercle et que la tige (12) est excentrée du côté dorsal.

7. Prothèse selon l'une des revendications 1 à 6, caractérisée en ce que l'élément de coque (21) qui forme la base de l'articulation d'un élément d'articulation distal (20) décrit sensiblement, en coupe longitudinale dorsale-palmaire (fig. 4), un quart de cercle et que la tige (22) est excentrée du côté palmaire.
